# EUROPÄISCHE PATENTSCHRIFT

(11) **EP 0 423 589 B1**
(45) Veröffentlichungstag und Bekanntmachung des Hinweises auf die Patenterteilung: **18.08.1993**
(21) Anmeldenummer: 90119300.3
(22) Anmeldetag: 09.10.1990
(51) Int. Cl.: A61B 17/068

(54) **Chirurgisches Gerät zum Setzen von Wundklammern**
Surgical stapler
Instrument d'agrafe chirurgical

(30) Priorität: 18.10.1989 DE 3934698
(43) Veröffentlichungstag der Anmeldung: 24.04.1991
(73) Patentinhaber: Beiersdorf Aktiengesellschaft, 20245 Hamburg (DE)
(72) Erfinder: Heimerl, Albert, Dr., W-2000 Hamburg 65 (DE); Kartheus, Holger, W-2000 Hamburg 20 (DE); Pietsch, Hans, Dr., W-2000 Hamburg 13 (DE); Voss, Bernd, W-2000 Norderstedt (DE)
(74) Vertreter: Wilcken, Thomas, Dipl.-Ing.

(56) Entgegenhaltungen:
- EP-A- 0 142 225
- EP-A- 0 284 345
- WO-A-83/00615

## Beschreibung

Die Erfindung geht aus von einem chirurgischen Gerät zum Setzen von Wundklammern, die im wesentlichen aus zwei Schenkeln und einem die Schenkel verbindenden Steg bestehen und die nacheinander aus einem Magazin auf ein Untergesenk bewegbar und dort mit einem Obergesenk verformbar sind, indem das Obergesenk aus einer Ruheposition in Richtung auf die jeweils zu setzende Klammer absenkbar und weiter verstellbar ist, um dabei die Klammer durch Biegen um das Untergesenk zu schließen und die Klammerschenkel in das Gewebe einzustechen, wobei ein Niederhalter beim Verformen der jeweils zu setzenden Klammer deren Steg in Richtung auf das Untergesenk drückt und einem Aufwölben des Steges entgegenwirkt.

Geräte dieser Art (EP 142 225 A1) und andere ähnliche Geräte (EP 040 683 A2, EP 284 345 A2, EP 244 854 A2, US-Patent 1 945 377 und US-Patent 3 873 016) werden insbesondere zum Verschließen von operativen Hautwunden eingesetzt. Mit ihnen wird heute in immer größer werdendem Umfang die traditionelle Methode des Nähens von Wunden ersetzt.

Man weiß allerdings auch, daß die Anwendung von Wundklammern auch gewisse Nachteile für die klinische Praxis mit sich bringt. Für die gute Heilung einer Hautnarbe ist es wichtig, daß die Wundheilung an der untersten Hautschicht beginnt. Deshalb stellt der Chirurg die Wundränder vor deren Verklammerung etwas nach oben auf und fixiert erst dann diese sogenannte Evertierung der Wundränder mit einer Klammernaht. Aufgrund einer mangelhaften Implantation der Wundklammern kann es aber dazu kommen, daß die Evertierungsposition nicht erhalten bleibt, so daß hieraus Treppennarben resultieren, die kosmetisch und auch bezüglich der Festigkeit der Narbe nachteilig sind.

Dieser Mangel kann einerseits auf eine ungünstige Form der Klammer, andererseits aber vor allem auf eine mangelhafte Funktion der bisher bekannten Wundklammergeräte zurückgeführt werden. Es hat sich nämlich gezeigt, daß die Klammern häufig unsymmetrisch verformt werden, obwohl sie in genau symmetrischer Ausrichtung im Klammermagazin bevorratet werden und auch die Gesenke keine erkennbare Unsymmetrie aufweisen.

Es wird zwei Gründe für eine unsymmetrische Klammerverformung und die damit verbundenen Nachteile geben, nämlich zum einen die beim Verformen bzw. Schließen der Klammer erfolgende Verschiebung des unteren, auf dem Untergesenk liegenden Randbereiches der Klammer zu dessen Mitte. Zum anderen zieht die zwangsläufig erfolgende Aufwölbung der Klammer im Bereich ihres die beiden seitlichen Klammerschenkel verbindenden Steges Klammermaterial nach innen, da das Obergesenk mit zwei nach unten auf die Klammer gerichteten Beinen beiderseits der Klammermitte auf die Klammer drückt und hierbei der Klammersteg unter Bildung der erwähnten Aufwölbung vom Untergesenk abgehoben wird.

Es kommt aus diesen Gründen an den beiden Berührungspunkten zwischen Klammer und Untergesenk zum Aufbau von auf die Klammer einwirkenden Zugkräften. Diese entgegengesetzt gerichteten Kräfte treten zunächst gleichmäßig links und rechts auf, aber nur so lange, bis die Zugkraft größer wird als die Haftreibungskraft am einen oder anderen Berührungspunkt. Dann wird nämlich die Klammer auf einer Seite in der betreffenden Berührungszone ins Gleiten kommen und weiter gleiten, da der Gleitreibungskoeffizient bekanntlich immer kleiner ist als der Haftreibungskoeffizient. Demzufolge wird die Klammer unter solchen Bedingungen immer mehr zu einer Seite hin gezogen und nicht symmetrisch implantiert, vor allem wenn dieses Verrutschen der Klammer auf dem Untergesenk noch von einer ungünstigen Krafteinleitung durch das Obergesenk in gleicher Richtung nachteilig beeinflußt wird. Im übrigen kann es bei einer unsymmetrischen Verformung der Klammer auch dazu kommen, daß sich die Klammer im Bereich der Verformungswerkzeuge verklemmt und nicht ohne weiteres freigegeben werden kann.

Als Klammermaterial wird im allgemeinen Stahldraht verwendet. Bei der Verformung bzw. Implantation wird eine Klammer aus solchem Material zwar überwiegend plastisch verformt, es liegt aber immer auch eine elastische Verformung vor, die beim Entlassen der Klammer aus den Gesenken dazu führt, daß sich die Klammer wieder etwas öffnet und aufspreizt. Diese Rückfederung hat insbesondere den Nachteil, daß die Wundrandadaptation nicht kontrollierbar ist.

Zur Lösung dieses Problems wurde schon vorgeschlagen (EP 284 345 A2), die Rückfederung in der Weise zu kompensieren, daß die Klammer beim Implantieren auch entgegen der eigentlichen Schließrichtung elastisch verformt und vorgespannt wird, so daß diese Vorspannung beim Freigeben der implantierten Klammer in Schließrichtung wirken und der unvermeidbaren Rückfederung entgegenwirken wird. Um dies zu erreichen, kann im Untergesenk eine konkave Mulde und am Obergesenk ein zur Mulde komplementär geformter konvexer Drucksteg vorgesehen werden, der beim Absenken des Obergesenkes den Klammersteg in die Mulde drücken wird.

Bei dieser vorbekannten Lösung befindet sich der Drucksteg am Obergesenk oberhalb der beiden seitlichen, ebenfalls zum Obergesenk gehörenden Beine, mit denen die Klammerschenkel durch entsprechendes Biegen der Klammer in das Gewebe gedrückt werden. Aufgrund dieser Konstruktion werden beim Absenken des Obergesenkes zunächst nur dessen Beine auf die Klammer einwirken und diese verformen, während der Drucksteg erst danach oder höchstens kurz vor dem Ende der eigentlichen Klammerverformung zur Wirkung kommen kann.

Deshalb wird sich bei dieser Lösung die vorher als nachteilig diskutierte Aufwölbung der Klammersteges nicht vermeiden lassen. Jedenfalls wird diese Aufwölbung erst aufgehoben, sobald der Drucksteg auf den Klammersteg gelangt und diesen in die Mulde preßt. Da hierbei die Klammer schon im wesentlichen ihre vorgesehene Verformung erreicht hat, wird außerdem Klammermaterial über die seitlichen Kanten des Untergesenkes in Richtung auf die Muldenmitte gezogen, was zur Folge haben kann, daß die Klammer wegen verschieden großer Haftreibungen an den Punkten ihrer Berührung mit dem Untergesenk unsymmetrisch verformt und zwischen den Gesenken eingeklemmt wird. Im übrigen ist es sehr fraglich, ob der Klammersteg überhaupt noch ausreichend elastisch vorgespannt werden kann, nachdem die Klammer schon im wesentlichen verformt und fest zwischen den Gesenken eingespannt ist.

Das Aufwölben der Klammer kann weitgehend vermieden werden, indem man oberhalb des Untergesenkes einen Niederhalter anordnet (EP 142 225 A1), gegen den der beim Verformungsvorgang nach oben ausweichende Klammersteg an einer Aufwölbung gehindert wird. Hiermit werden aber nicht die vorher aufgezeigten Probleme gelöst, weil die Klammer bzw. deren Steg von der Seite her mit ausreichend Spiel in den Raum zwischen Untergesenk und Niederhalter eingeführt werden muß, was bedeutet, daß ein seitliches Verschieben und somit eine unsymmetrische Verformung der Klammer auf dem Untergesenk nicht wirksam verhindert werden können, weil der Niederhalter erst gegen den Klammersteg zur Anlage kommt und wirksam wird, nachdem die Klammer schon teilweise verformt und möglicherweise bereits seitlich versetzt ist.

Die Aufgabe der Erfindung besteht in der Schaffung eines mechanisch sicher funktionierenden Wundklammergerätes, welches insbesondere eine absolut symmetrische Verformung und Implantation der Klammern gewährleistet. Weiterhin soll der Effekt der elastischen Rückfederung der Klammer weitestgehend kompensiert werden.

Zur Lösung dieser Aufgabe wird vom eingangs erwähnten Gerät ausgegangen und dieses erfindungsgemäß so ausgebildet, daß beim Betätigen des Gerätes zunächst der Niederhalter und anschließend das Obergesenk zur Anlage mit der Klammer bringbar sind. Hierdurch wird der Klammersteg stets unter Druck auf dem Untergesenk gehalten und ausgeschlossen, daß sich der Steg beim Verformen aufwölbt und seitlich auf dem Untergesenk verschoben werden kann.

Das Obergesenk wird zweckmäßigerweise zusammen mit dem Niederhalter aus der erwähnten Ruheposition bis in eine Zwischenposition verstellt, bei der sich zunächst nur der Niederhalter in Anlage mit dem Klammersteg befindet, während das Obergesenk unter Spannen einer zwischen ihm und dem Niederhalter wirkenden Federeinrichtung allein weiter verstellbar ist, um die Klammer zu verformen.

Zur Kompensation des elastischen Rückfederns und Aufspreizens der Klammer beim Entlassen aus den Gesenken wird das Gerät so ausgebildet, daß das Untergesenk eine konkave Mulde hat und daß das auf diese Mulde ausgerichtete Ende des Niederhalters einen konvexen Verlauf hat, derart, daß die Klammer im Bereich des Steges durch den Niederhalter eine elastische Verformung erfährt, die der Klammerschließrichtung entgegengesetzt ist.

Da beim Betätigen des Gerätes zunächst der Niederhalter und dann erst das Obergesenk zur Anlage gegen die Klammer gelangen soll, kann die vorerwähnte elastische Verformung des Klammersteges durch dessen Eindrücken in die Mulde mittels des Niederhalters erfolgen, bevor die Klammer mit dem dann weiter zu verstellenden Obergesenk verformt und implantiert wird. Es kann also nicht dazu kommen, daß Klammermaterial über die seitlichen Kanten des Untergesenkes zur Muldenmitte hin gezogen und die Klammer unsymmetrisch verformt wird, zumal hierbei der Niederhalter den elastisch verformten Stegteil in der Mulde des Untergesenkes gegen Aufwölben und seitliches Verschieben fixiert.

Das zeitlich versetzte Auftreffen von Niederhalter und Obergesenk auf die Klammer ist insofern also die beste Lösung, obwohl es zu erwarten und nicht auszuschließen ist, daß wenigstens annähernd gleich gute Ergebnisse erzielt werden können, wenn der Niederhalter und das Obergesenk beim Absenken in Richtung auf das Untergesenk annähernd gleichzeitig auf die Klammer treffen würden.

Unterstützt wird die einwandfreie Fixierung der Klammer auf dem Untergesenk noch, wenn das Untergesenk im Verhältnis zu dem Schlitten, auf dem eine Reihe von Klammern gleitend verschiebbar liegt, nach unten abgesetzt ist und wenn die Höhe h des Absatzes und der Durchmesser d des Klammermaterials in folgender Beziehung zueinander stehen, nämlich 0,25d ≦ h ≦ d. Der Absatz wird nämlich die zu verformende Klammer auch noch an der hinteren Seite ihres Steges wirksam gegen Abkippen stützen und führen.

Bei bekannten Geräten ist im allgemeinen ein erster Griff mit einem das Magazin, das Obergesenk und weitere Geräteteile aufnehmenden Gehäuse starr verbunden, während ein zweiarmiger Hebel , dessen längerer Arm einen zweiten Griff bildet, um eine Achse gegen und mit Federwirkung relativ zum Gehäuse und ersten Griff verschwenkt werden kann, um hierbei mit seinem kürzeren Hebelarm das Obergesenk zu verstellen.

Ein Gerät mit solchen Merkmalen wird nach der Erfindung vorteilhaft in der Weise weiter ausgestaltet, daß das Obergesenk und der Niederhalter mit fluchtenden Öffnungen versehen sind, durch die der kürzere Arm des Hebels greift, daß das Obergesenk und der Niederhalter mit seitlich abstehenden Laschen versehen sind, zwischen denen eine Druckfeder eingespannt ist, welche das Obergesenk und die Niederhalter in einer durch einen Anschlag festgelegten Lage zueinander hält, und daß beim Zusammendrücken der Griffe der kurze Hebelarm auf die am Obergesenk befindliche Lasche einwirkt und das Obergesenk sowie die Niederhalter in Richtung auf das Untergesenk verstellt, und zwar zunächst gemeinsam bis zur erwähnten Zwischenposition und anschließend allein weiter das Obergesenk relativ zum dann feststehenden Niederhalter. Hierdurch ergibt sich eine einfache und sicher funktionierende Ausführungsform für das Obergesenk und den Niederhalter sowie für die zu deren Verstellung erforderlichen Teile.

Die Erfindung wird nachfolgend anhand eines in der Zeichnung dargestellten Ausführungsbeispiels näher erläutert. Es zeigt bzw. zeigen:
- Figur 1: die Seitenansicht eines erfindungsgemäßen Gerätes in teilweisem Schnitt,
- Figur 2: einen Schnitt des Gerätes nach Figur 1 entsprechend der Schnittlinie II - II,
- Figur 3: die Vorderansicht des Gerätes gemäß Fig. 1,
- Figur 4: einen Schnitt durch den vorderen Teil des Gerätes entsprechend der Schnittlinie IV - IV in Fig. 2,
- Figur 5: den Niederhalter und das Obergesenk in Rückansicht und in zwei verschiedenen, einander parallel gegenübergestellten Positionen relativ zum Untergesenk,
- Figuren 6 u. 7: Seitenansichten des Niederhalters und des Obergesenkes im Längsschnitt und in verschiedenen Positionen,
- Figuren 8 - 11: verschiedene Stellungen der Verformungswerkzeuge und
- Figur 12: eine im wesentlichen der Figur 1 entsprechende Darstellung des Gerätes in getrenntem Zustand.

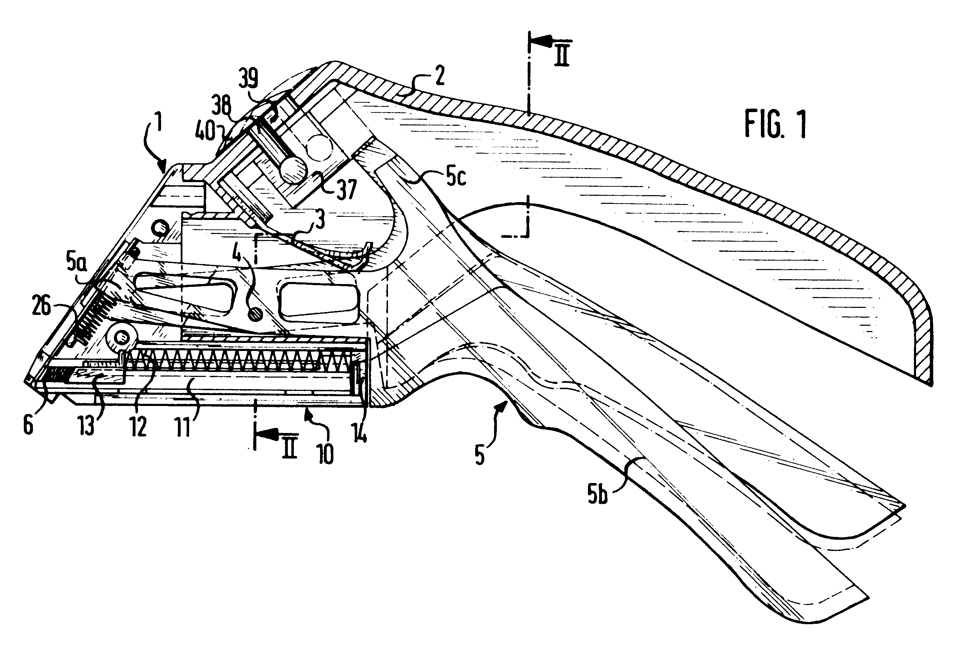

Das Gerät besteht aus einem Gehäuse 1, das mit einem Griff 2 verbunden ist. Relativ zu diesem Griff ist gegen die Wirkung einer am Gehäuse 1 befestigten Feder 3 ein zweiarmiger Hebel⁵/von Hand um die Achse 4 verschwenkbar. Der Hebel besteht aus einem kurzen Hebelarm 5a und einem längeren Hebelarm 5b, der einen zweiten Griff bildet. Im übrigen werden beide Griffe mit Wirkung der Feder 3 in der dargestellten Position nach Fig. 1 auf Abstand gehalten.

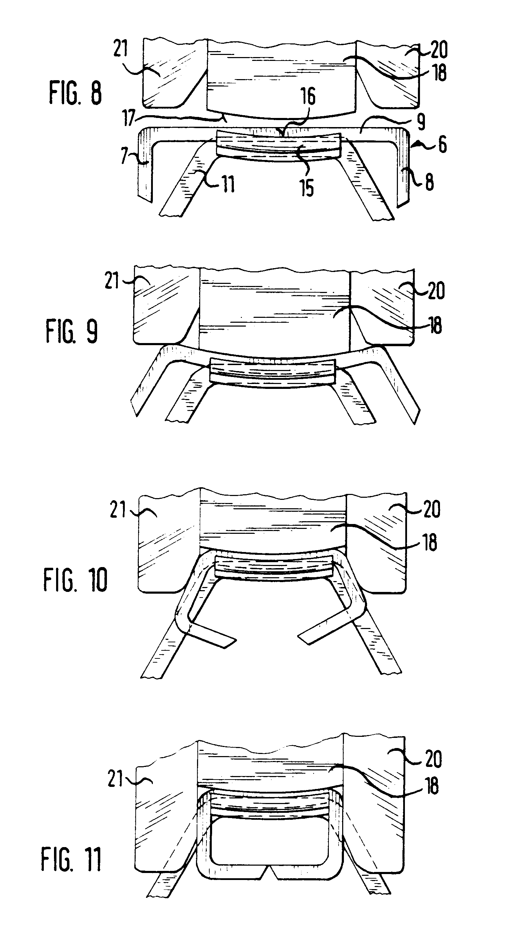

Die Wundklammern 6, die aus zwei mit Schneiden bzw. Spitzen versehenen Schenkeln 7,8 und einem diese Schenkel verbindenden Steg 9 bestehen (Figur 8), sind in einem Magazin 10 hintereinander in Form einer Klammerreihe bevorratet. Das Magazin 10 besteht aus einem Schlitten 11, auf dem die Klammern 6 gleitend in Richtung auf das distal offene Ende des Gehäuses 1 in den Wirkungsbereich der später beschriebenen Verformungswerkzeuge verschiebbar sind, und zwar mit Hilfe einer Druckfeder 12,die mit einem Ende gegen einen frei auf dem Schlitten 11 gleitenden Schuh 13 drückt und am anderen Ende fest am Teil 14 abgestützt ist.Wie aus Figur 1 ersichtlich ist, drückt also der Schuh 13 die Klammerreihe in die vorerwähnte Richtung.

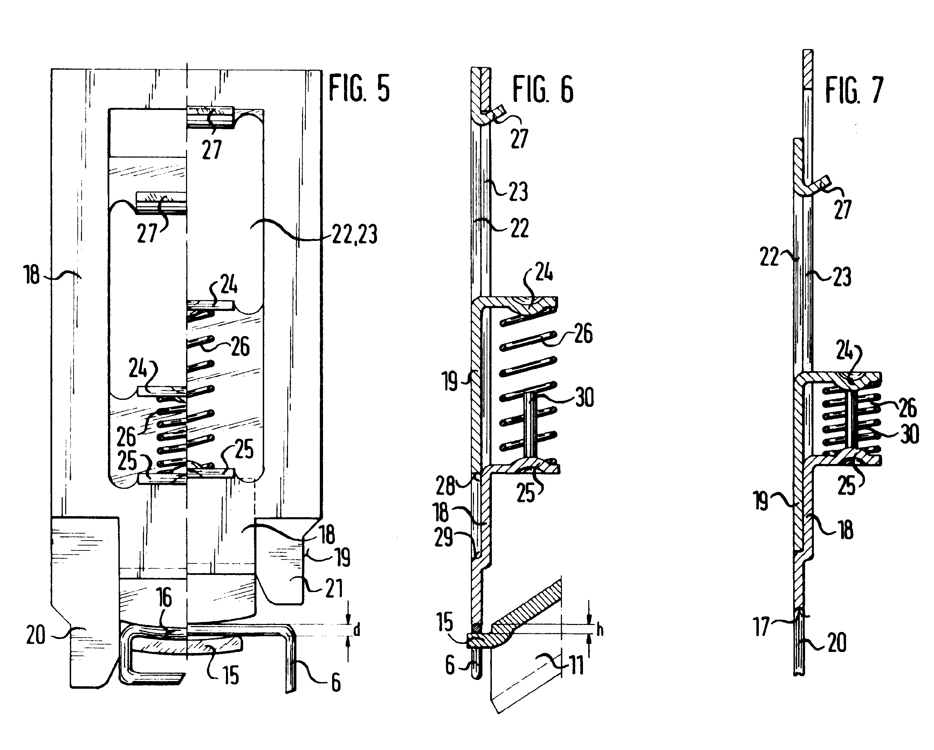

Am Schlitten 11 ist ein Untergesenk 15 angeformt. Dieses ist im Verhältnis zum benachbarten Ende des Schlittens 11 nach unten abgesetzt (Figur 6), so daß sich die jeweils vordere Klammer 6 vor ihrer Verformung zunächst mit Abstand über dem Untergesenk 15 befindet und erst beim Betätigen des Gerätes durch Einsatz von Kraft auf das Untergesenk bewegt wird.

Die Höhe h des Absatzes und der Durchmesser d des Klammermaterials stehen zweckmäßigerweise in folgender Beziehung zueinander, nämlich 0,25 d ≦ h ≦ d. Diese Verhältnisse sind insofern günstig, als hierdurch zum einen eine ausreichende hintere Abstützung und Führung der auf das Untergesenk zu bewegenden Klammer erreicht und zum anderen ein unnötig langer Weg für diese Klammerbewegung vermieden wird.

Insbesondere die Figuren 5 und 8 bis 11 lassen erkennen, daß das Untergesenk 15 eine quer über seine Breite verlaufende konkave Mulde 16 hat, auf die das konvexe Ende 17 eines Niederhalters 18 ausgerichtet ist, damit aus einleitend erwähnten Gründen die jeweilige Klammer 6 im Bereich ihres Steges 9 durch den in Richtung auf das Untergesenk 15 und die Klammer abgesenkten Niederhalter 18 eine elastische Verformung erfahren wird (Figur 9), die der eigentlichen Schließrichtung der Klammer entgegengerichtet ist. Die Radien des konvexen Niederhalterendes 17 und der Mulde 16 sind im wesentlichen gleich groß und liegen je nach Klammerform und Klammermaterial in der Größenordnung von 60 bis 80 mm, vorzugsweise in der Größenordnung von 65 bis 75 mm, wobei der Radius des Niederhalterendes 69 mm und der Radius der Mulde 70 mm betragen kann. Im übrigen werden die Radien innerhalb der vorerwähnten Größenordnung im allgemeinen umso kleiner gewählt, je höhe die Festigkeit bzw. Streckgrenze des Klammermaterials ist.

Der Niederhalter 18 und das mit ihm zusammenwirkende Obergesenk 19 sind durch Stanzen und Prägen aus Blech hergestellte Bauteile, wobei für das Schließen der Klammer in bekannter Weise die beiden nach unten abstehenden Beine 20,21 vorgesehen sind, die beim Absenken des Obergesenkes 19 das Untergesenk 15 seitlich passieren (Figuren 10 und 11).

Das untere Ende 17 des Niederhalters 18 und die beim Verformungsvorgang wirksamen Enden der Beine 20, 21 sollten ausgekehlt sein (Figur 7), damit die Klammer beim Schließen bzw. Implantieren sicher duch Formschluß in den Auskehlungen gehalten wird und nicht nach hinten oder vorn kippen kann.

Das Obergesenk 19 und der Niederhalter 18 haben fluchtende Öffnungen bzw. Ausschnitte 22,23, durch die der Hebelarm 5a mit seinem freien Ende greift. Weiterhin sind das Obergesenk 19 und der Niederhalter 18 mit jeweils einer seitlich nach hinten abstehenden Lasche 24 bzw. 25 versehen, zwischen denen eine Druckfeder 26 eingespannt ist, die bei unbetätigtem Gerät das Obergesenk und die Niederhalter in einer festgelegten Lage zueinander hält (Figur 6). Diese Lage wird durch den am Obergesenk 19 ausgebildeten Anschlag 27 bestimmt, der als Haken durch die Öffnung 23 des Niederhalters 18 greift und gegen den oberen inneren Rand dieser Öffnung anliegt.

Beim Zusammendrücken der beiden Griffe 2,5b wird der Hebelarm 5a auf die Lasche 24 Druck ausüben, so daß das Obergesenk 19 und der Niederhalter 18 zunächst gemeinsam aus einer oberen Ruheposition heraus nach unten bis in eine Zwischenposition (Fig. 6) verstellt werden, ohne daß sie dabei relativ zueinander verstellt werden. Zunächst trifft also das Ende 17 des Niederhalters 18 auf den Steg 9 der jeweils vorderen, aus dem Magazin kommenden Klammer 6 und schiebt diese nach unten bis auf das Untergesenk 15.

Durch weiteres und ggf. stärkeres Zusammendrücken der Griffe 2,5b preßt der Niederhalter 18 den Klammersteg 9 unter möglichst nur elastischer Verformung des Klammersteges in die Mulde 16 (Figur 9), wobei zu diesem Zweck die Feder 26 natürlich so zu dimensionieren ist, daß hierbei nicht auch schon das Obergesenk 19 relativ zum Niederhalter nach unten verstellt wird, obwohl insofern auch eine solche Verstellbewegung in geringem Maße zugelassen werden kann, sofern gewährleistet bleibt, daß bei diesem Vorgang die Beine 20,21 des Obergesenkes 19 nicht schon zur Anlage mit der Klammer 6 gelangen und daß elastische Aufspreizen der Klammer nicht behindern.

Eine weitere Bewegung des Niederhalters 18 nach unten ist nach dem Erreichen der Verformungsphase gemäß Fig. 9 nicht möglich, so daß der von oben auf die Lasche 24 drückende Hebelarm 5a allein das gleitend am Niederhalter 18 geführte Obergesenk 19 weiter nach unten verstellen wird, wobei schließlich das Obergesenk mit seinen Beinen 20,21 auf die Klammerschenkel 7,8 trifft (Fig.9) und diese zum Schließen der Klammer durch Biegen um die seitlichen Kanten des Untergesenkes 15 verformt (Figuren 10 und 11).

Hierbei wird die Feder 26 kontinuierlich stärker zusammengedrückt.Der insofern größtmögliche Federweg und auch die größtmögliche Verstellbewegung des Obergesenkes 19 relativ zum Niederhalter 18 nach unten können in der Weise festgelegt werden, daß eine Kante 28 am Obergesenk und ein Absatz 29 am Niederhalter vorgesehen werden, die Anschläge bilden und beim Erreichen einer Endposition gemäß Fig. 7 gegenseitig zur Anlage gelangen.

Zusätzlich oder alternativ kann die erwähnte Verstellbewegung auch mit einem zwischen Obergesenk und Niederhalter vorgesehenen Distanzstift 30 begrenzt werden, der beispielsweise stehend auf der Lasche 15 befestigt ist (Figur 6) und dessen freies Ende als Anschlag von unten gegen die Lasche 24 stößt, sobald die Endposition nach Fig. 7 erreicht wird.

Nach Öffnen der die Griffe 2,5b umfassenden Hand kann die Feder 3 den Hebel 5 wieder in die Ausgangsposition gemäß Fig.1 bringen, wobei gleichzeitig der im Uhrzeigersinn verschwenkte Hebelarm 5b, auch unter Mitwirkung der sich entspannenden Feder 26, den Niederhalter 18 und das Obergesenk 19 nach oben in die Ruheposition verstellt,so daß die implantierte Klammer 6 einfach durch Abstreifen vom Untergesenk 15 aus dem Gerät entlassen werden kann.

Sobald das Obergesenk durch Verstellung nach oben außer Kontakt mit der Klammer gelangt, neigt die Klammer dazu, sich teilweise wieder zu öffnen, weil sie mit dem Obergesenk auch elastisch in Schließrichtung verformt wurde.Diesem Aufspreizen der Klammer wirkt allerdings die mit dem Niederhalter bewirkte elastische Verformung der Klammer entgegen, so daß das nachteilige Aufspreizen der Klammer wenigstens weitgehend kompensiert wird.

Abschließend wird noch darauf hingewiesen, daß das Magazin mit Klammern und auch sonstige Teile, wie etwa Niederhalter, Obergesenk und ein Teil des Gehäuses,zu einer gesonderten baulichen Einheit zusammengefaßt werden können, die als Artikel für den einmaligen Gebrauch lösbar durch Aufstecken am verbleibenden Gehäuseteil befestigt werden kann. Die sonstigen Geräteteile sollten sterilisierbar und aus Kostengründen mehrfach verwendbar sein.

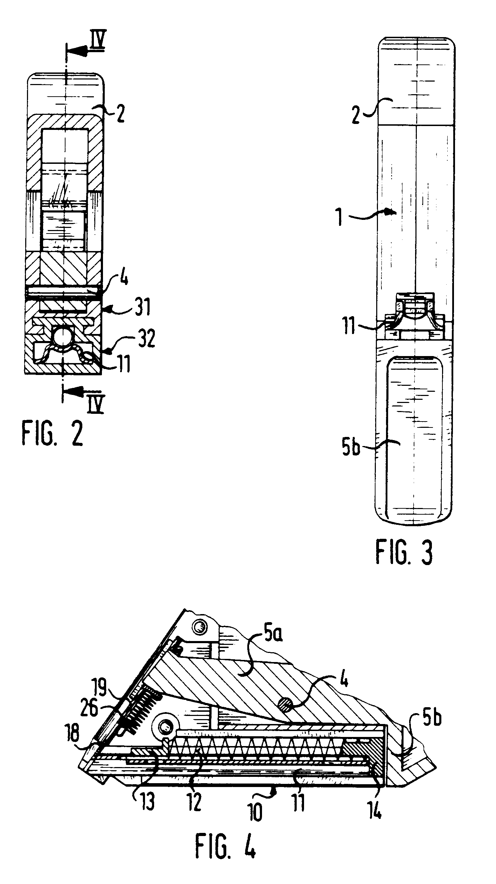

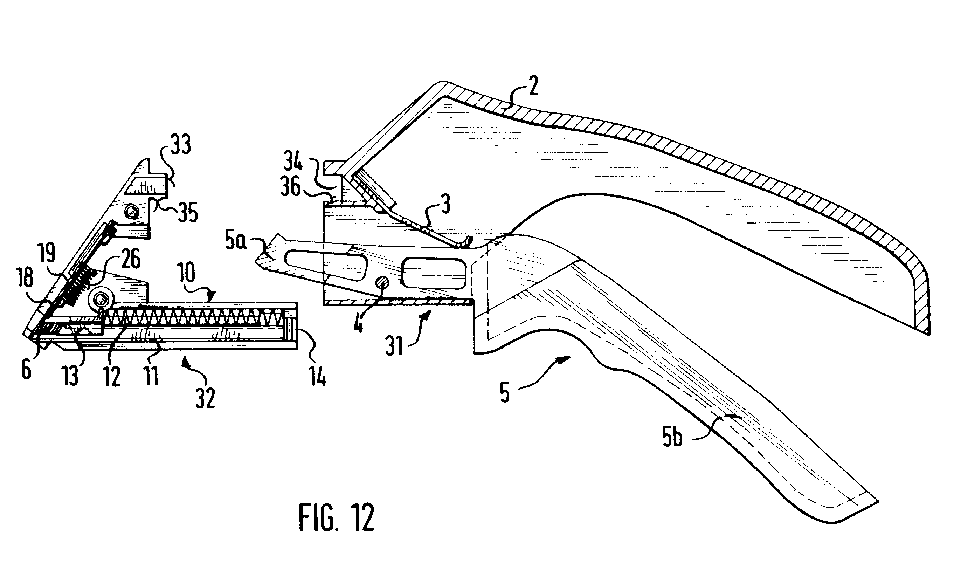

Eine insofern besonders geeignete Lösung zeigt die Figur 12 mit dem nach Gebrauch wiederverwendbaren ersten Geräteteil 31 und dem hiervon abgezogenen, auswechselbaren zweiten Geräteteil 32 für den einmaligen Gebrauch.Aus der Figur 2 ist ersichtlich, daß beide Teile 31,32 im Bereich oberhalb des Schlittens 11 formschlüssig ineinandergreifende Führungen haben, die ein geführtes Zusammenfügen und Lösen der Teile 31,32 ermöglichen. Weiterhin ist am Teil 32 oben ein Ansatz 33 vorgesehen, der beim Fügen der beiden Geräteteile in eine Aufnahme 34 des Teiles 31 greift, wobei zur Schaffung einer Rastverbindung eine am Ansatz 33 befindliche Nase 35 in eine Ausnehmung 36 der Aufnahme 34 schnappen wird.

Im übrigen macht es diese Lösung erforderlich, daß das Gerätegehäuse zweiteilig ausgebildet wird und daß das Ende des kurzen Hebelarmes 5a frei durch die Öffnungen 22,23 des Obergesenkes und des Niederhalters greift, also mit diesen nicht fest verbunden ist.

Der auswechselbare Geräteteil 32 umfaßt der Fig. 12 entsprechend insbesondere folgende Teile, nämlich das Magazin 10 mit dem Schlitten 11 und dem Untergesenk 15, die Druckfeder 12, den Schuh 13 und die Klammer 6 sowie das Obergesenk 19 und den Niederhalter 18 mit der zwischen ihnen wirkenden Feder 26 und schließlich den vorderen Teil des Gerätegehäuses 1. Wesentliche Bauteile des wiederverwendbaren Geräteteiles 31 sind der doppelarmige Hebel 5, der Griff 2, die Achse 4, die Feder 3 und der hintere Teil des Gerätegehäuses 1.

Von besonderer Bedeutung ist es bei dieser Lösung, daß u.a. die Gesenke als klammerverformende Bauteile mit zum auswechselbaren Teil 32 gehören. Bei anderen bekannten Geräten (DE 36 15 405 A1),bei denen meist nur das Magazin und ggf. auch hiermit noch das Untergesenk auswechselbar sind, gehört das Obergesenk zum mehrfach zu verwendenden Geräteteil, bleibt also stets direkt mit dem kraftausübenden Hebel zum Verstellen des Obergesenkes verbunden.

Das hat den Nachteil, daß die Gesenke nach Ersatz des Wegwerfteiles durch ein neues häufig nicht mehr exakt zusammenpassen, zumal auch am Obergesenk mit der Zeit ein gewisser Verschleiß auftreten wird. Dies wird zu einer mangelhaften Klammerverformung führen, was dagegen nicht passieren wird,wenn mit dem Magazin jeweils auch beide Gesenke mit ausgetauscht werden.

Insbesondere für die Wundrandverbindung im Bereich großer Gewebeschwellungen und auch zur Vermeidung übermäßiger Verletzungen der Unterhaut, wie etwa bei der Fixierung von Hauttransplantaten,wird eine variable Wundrandadaptierung von Vorteil sein. Hierbei wird die Klammer nicht vollständig wie in Figur 11 gezeigt verformt, sondern nur teilweise geschlossen, wie es beispielsweise die Figur 10 zeigt.

Zu diesem Zweck sollte das jeweils gewünschte Maß der Klammerverformung mit einem herstellbaren Anschlag variabel eingestellt werden können, wobei dieser Anschlag die maximal mögliche Verstellbewegung eines der Handgriffe begrenzt.

Hierfür zeigt die Figur 1 eine von mehreren geeigneten Lösungen. Der betreffende Anschlag 37 steht über einen Steg 38, der durch ein Langloch 39 des hinteren Gehäuseteiles greift, mit einem von außen zugänglichen Schieber 40 in Verbindung. Am Hebel 5 ist ein Fortsatz 5c angeformt, der bei der in ausgezogenen Linien dargestellten Position des Anschlages 37 bzw. des Schiebers 40 beim Schwenken des Griffes 5b nach oben den Anschlag 37 frei passieren kann, so daß in diesem Fall ungehindert eine vollständige Verformung der Klammer möglich ist.

Wenn allerdings durch Betätigung des Schiebers 40 dieser und damit auch der Anschlag 37 der Darstellung entsprechend schräg nach oben in die gestrichelt gezeigte Position verstellt sind, wird beim Zusammendrücken der Griffe 2,5b das freie Ende des Fortsatzes 5c auf den ihm dann gegenüberliegenden Anschlag 37 treffen, wobei sowohl eine weitere Schwenkbewegung des Hebels 5 als auch eine weitere Verstellbewegung des Obergesenkes 19 nach unten verhindert werden mit der Folge, daß die jeweilige Klammer zwangsläufig nicht vollständig verformt werden kann.

## Patentansprüche

1. Chirurgisches Gerät zum Setzen von Wundklammern (6), die im wesentlichen aus zwei Schenkeln (7, 8) und einem die Schenkel verbindenden Steg (9) bestehen und die nacheinander aus einem Magazin (10) auf ein Untergesenk (15) bewegbar und dort mit einem Obergesenk (19) verformbar sind, indem das Obergesenk (19) aus einer Ruheposition in Richtung auf die jeweils zu setzende Klammer (6) absenkbar und weiter verstellbar ist, um dabei die Klammer (6) durch Biegen um das Untergesenk (15) zu schließen und die Klammerschenkel (7, 8) in das Gewebe einzustechen, wobei ein Niederhalter (18) beim Verformen der jeweils zu setzenden Klammer (6)deren Steg (9) in Richtung auf das Untergesenk (15) drückt und einem Aufwölben des Steges (9) entgegenwirkt, dadurch gekennzeichnet, daß beim Betätigen des Gerätes zunächst der Niederhalter (18) und anschließend das Obergesenk (19) zur Anlage mit der Klammer (6) bringbar sind.

2. Gerät nach Anspruch 1, dadurch gekennzeichnet, daß das Untergesenk (15) eine konkave Mulde(16) hat und daß das auf diese Mulde ausgerichtete Ende (17) des Niederhalters (18) einen konvexen Verlauf hat, derart, daß die Klammer (6) im Bereich des Steges (9) durch den Niederhalter eine elastische Verformung erfährt, die der Klamerschließrichtung entgegengesetzt ist.

3. Gerät nach Anspruch 2, dadurch gekennzeichnet, daß die Radien des konvexen Niederhalterendes (17) und der Mulde (16) im wesentlichen gleich groß sind und in der Größenordnung von 60 bis 80 mm, insbesondere 65 bis 75 mm liegen.

4. Gerät nach einem der Ansprüche 1 bis 3, dadurch gekennzeichnet, daß das Obergesenk (19) mit dem Niederhalter (18) aus der erwähnten Ruheposition bis in eine Zwischenposition verstellbar ist, bei der sich der Niederhalter in Anlage mit dem Klammersteg (9) befindet, und daß das Obergesenk unter Spannen einer zwischen ihm und dem Niederhalter wirkenden Federeinrichtung (26) weiter verstellbar ist, um die Klammer (6) zu verformen.

5. Gerät nach Anspruch 4, bei dem ein erster Griff mit einem das Magazin ,das Obergesenk und weitere Geräteteile aufnehmenden Gehäuse starr verbunden ist und bei dem ein zweiarmiger Hebel vorgesehen ist, dessen längerer Arm einen zweiten Griff bildet und der um eine Achse gegen und mit Federwirkung relativ zum Gehäuse und ersten Griff verschwenkbar ist, um mit seinem kürzeren Arm das Obergesenk zu verstellen,dadurch gekennzeichnet, daß das Obergesenk (19) und der Niederhalter(18) mit fluchtenden Öffnungen (22,23) versehen sind, durch die der kürzere Arm (5a) des Hebels (5) greift, daß das Obergesenk und der Niederhalter mit seitlichen abstehenden Laschen (24,25) versehen sind, zwischen denen eine Druckfeder (26) eingespannt ist, welche das Obergesenk und den Niederhalter in einer durch einen Anschlag (27) festgelegten Lage zueinander halt, und daß beim Zusammendrücken der Griffe (2,5b)der kurze Hebelarm (5a)auf die am Obergesenk befindliche Lasche (24) einwirkt und das Obergesenk sowie den Niederhalter in Richtung auf das Untergesenk (15) verstellt, und zwar zunächst gemeinsam bis zur erwähnten Zwischenposition und anschließend allein weiter das Obergesenk relativ zum dann feststehenden Niederhalter.

6. Gerät nach einem der Ansprüche 1 bis 5, dadurch gekennzeichnet, daß die größtmögliche Verstellbewegung des Obergesenkes (19) relativ zum Niederhalter (18)nach unten durch Anschläge (28,29;24,30) begrenzt ist.

7. Gerät nach einem der Ansprüche 1 bis 6, bei dem eine Reihe von Klammern gleitend verschiebbar auf einem Schlitten liegt, dadurch gekennzeichnet, daß das Untergesenk (15) im Verhältnis zum Schlitten (11) nach unten abgesetzt ist und daß die Höhe h des Absatzes und der Durchmesser d des Klammermaterials in folgender Beziehung zueinander stehen, nämlich 0,25d ≦ h ≦ d.

8. Gerät nach einem der Ansprüche 1 bis 7, bestehend aus einem ersten Geräteteil für den mehrfachen Gebrauch und einem lösbar mit diesem verbundenen, das Magazin umfassenden zweiten Geräteteil für den einmaligen Gebrauch, dadurch gekennzeichnet, daß dem zweiten Geräteteil (32) außer dem Magazin (10) auch die beiden Gesenke (15,19) und der Niederhalter (18) zugeordnet sind.

9. Gerät nach Anspruch 8, dadurch gekennzeichnet, daß die beiden Geräteteile (31,32) mittels einer Rastverbindung (35,36) zusammengehalten werden.

10. Gerät nach einem der Ansprüche 1 bis 9, dadurch gekennzeichnet, daß das Maß der Klammerverformung mit einem verstellbaren Anschlag (37), insbesondere mit einem die Verstellbewegung des verschwenkbaren Griffes (5b)begrenzenden Anschlag, variabel einstellbar ist.

## Claims

1. A surgical instrument for the installing of wound clamps (6), which consist substantially of two shanks (7, 8) and a cross-piece (9) connecting the shanks, and which are movable one after the other from a magazine (10) onto a lower die (15) and are able to be formed there with an upper die (19), in which the upper die (19) is able to be lowered from a position of rest in the direction of the clamp (6) which is to be installed in each case and is further adjustable in order to thereby close the clamp (6) by bending the clamp shanks (7, 8) around the lower die (15) to pierce the tissue, in which a holding-down device (18) on forming of the clamp (6), which is to be installed in each case, presses its cross-piece (9) in the direction of the lower die (15) and counteracts a bulging of the cross-piece (9), characterised in that on operation of the instrument, firstly the holding-down device (18) and then the upper die (19) are able to be brought to abutment with the clamp (6).

2. An instrument according to claim 1, characterised in that the lower die (15) has a concave depression (16) and that the end (17) of the holding-down device (18), aligned with this depression, has a convex shape, such that the clamp (6) in the region of the cross-piece (9) undergoes an elastic deformation through the holding down device, which deformation is opposed to the closing direction of the clamp.

3. An instrument to claim 2, characterised in that the radii of the convex end (17) of the holding-down device and of the depression (16) are of substantially equal size and lie in the order of 60 to 80mm, in particular 65 to 75mm.

4. An instrument according to one of claims 1 to 3 characterised in that the upper die (19) with the holding down device (18) is able to be displaced from the above-mentioned position of rest into an intermediate position, in which the holding-down device is in abutment with the clamp cross-piece (9) and that the upper die, under tensioning of a spring arrangement (26) acting between it and the holding-down device, is further displaceable, in order to deform the clamp (6).

5. An instrument according to claim 4, in which a first handle is rigidly connected with a housing holding the magazine, the upper die and further parts of the instrument, and in which a two armed lever is provided, the longer arm of which forms a second handle and which is orientable about an axis against and with spring action relative to the housing and first handle, in order to displace the upper die with its shorter arm, characterised in that the upper die (19) and the holding-down device (18) are provided with openings (22, 23) in alignment, through which the shorter arm (5a) of the lever (5) engages, that the upper die and the holding-down device are provided with laterally projecting tongues (24, 25), between which a compression spring (26) is fixed, which holds the upper die and the holding-down device to each other in a position fixed by a stop (27), and that on pressing together the handles (2, 5b), the short lever arm (5a) acts on the tongue (24) situated on the upper die and moves the upper die and also the holding-down device in the direction of the lower die (15), firstly together to the above mentioned intermediate position and then the upper die alone further relative to the holding-down device, which is then stationary.

6. An instrument according to claims 1 to 5, characterised in that the greatest possible downward displacement movement of the upper die (19) relative to the holding-down device (18) is delimited by stops (28, 29; 24, 30).

7. An instrument according to one of claims 1 to 6, in which a series of clamps lie so as to be slidingly displaceable on a sliding carriage, characterised in that the lower die (15) is offset downwards in relation to the sliding carriage (11) and that the height h of the step and the diameter d of the clamp material are in the following relationship to each other, namely 0.25d ≦ h ≧ d.

8. An instrument according to one of claims 1 to 7, consisting of a first instrument part for multiple use and of a second instrument part, detachably connected therewith, including the magazine, for once-only use, characterised in that there are also associated with the second instrument part (32), apart from the magazine (10), the two dies (15, 19) and the holding-down device (18).

9. An instrument according to claim 8, characterised in that the two instrument parts (31, 32) are held together by means of a detent connection (35, 36).

10. An instrument according to one of claims 1 to 9, characterised in that the extent of the clamp deformation is able to be variably adjusted with a displaceable stop (37), in particular with a stop delimiting the displacement movement of the orientable handle (5b).

## Revendications

1. Instrument chirurgical de pose d'agrafes chirurgicales (6), qui se composent essentiellement de deux branches (7, 8) et d'une entretoise (9) reliant les deux branches et peuvent être sorties l'une après l'autre d'un magasin (10) sur une matrice inférieure (15) et y être déformées par une matrice supérieure (19), la matrice supérieure (19) pouvant être abaissée à partir d'une position de repos en direction de l'agrafe respective à poser et pouvant être déplacée au-delà, afin de fermer ainsi l'agrafe (6) par un pliage autour de la matrice inférieure (15) et d'enfoncer dans le tissu les branches (7, 8) de l'agrafe, un dispositif (18) de maintien en position basse appuyant, lors de la déformation de l'agrafe respective à poser (6), l'entretoise (9) de celle-ci en direction de la matrice inférieure (15) et s'opposant à une courbure de l'entretoise (9),
caractérisé en ce que le dispositif (18) de maintien en position basse en premier lieu, et ensuite la matrice supérieure (19) peuvent être amenés en appui sur l'agrafe (6) lors de l'actionnement du dispositif.

2. Instrument selon la revendication 1, caractérisé en ce qu'une auge concave (16) est ménagée dans la matrice inférieure (15) et en ce que le tracé de l'extrémité (17), orientée vers cette auge, du dispositif (18) de maintien en position basse est convexe de sorte que l'agrafe (6) est soumise par le dispositif (18) de maintien en position basse à une déformation élastique dont le sens est opposé à la direction de fermeture de l'agrafe.

3. Instrument selon la revendication 2, caractérisé en ce que les rayons de l'extrémité convexe (17) du dispositif de maintien en position basse et de l'auge (16) sont sensiblement égaux et que leur ordre de grandeur est compris entre 60 et 80 mm, en particulier entre 65 et 75 mm.

4. Instrument selon l'une des revendications 1 à 3, caractérisé en ce que la matrice supérieure (19) peut être déplacée avec le dispositif (18) de maintien en position basse depuis la position de repos mentionnée vers une position intermédiaire dans laquelle le dispositif de maintien en position basse s'appuie sur l'entretoise (9) d'agrafe, et en ce que la matrice supérieure peut être déplacée au-delà en sollicitant un dispositif élastique (26) agissant entre elle et le dispositif de maintien en position basse afin de déformer l'agrafe (6).

5. Instrument selon la revendication 4, dans lequel une première poignée est reliée de façon rigide à un boîtier recevant le magasin, la matrice supérieure et d'autres parties de l'instrument et dans lequel est prévu un levier à deux bras dont le bras le plus long constitue une deuxième poignée et qui peut pivoter autour d'un axe contre le boîtier et la première poignée, en étant sollicité élastiquement par rapport à eux, afin de déplacer la matrice supérieure par son bras plus court, caractérisé en ce qu'il est ménagé, dans la matrice supérieure (19) et le dispositif (18) de maintien en position basse, des ouvertures alignées (22, 23) à travers lesquelles passe le bras plus court (5a) du levier (5), en ce que la matrice supérieure et le dispositif de maintien en position basse sont pourvus d'attaches latérales espacées (24, 25) entre lesquelles est sollicité un ressort de compression (26) qui maintient la matrice supérieure et le dispositif de maintien en position basse dans une position fixe l'un par rapport à l'autre déterminée par une butée (27), et en ce que le bras court (5a) du levier agit, lorsqu'il est rapproché par une pression sur les poignées (2, 5b), sur l'attache (24) située sur la matrice supérieure, et déplace d'abord en commun la matrice supérieure ainsi que le dispositif de maintien en position basse en direction de la matrice inférieure (15) jusqu'à la position intermédiaire mentionnée, et ne déplace ensuite, au-delà, que la matrice supérieure par rapport au dispositif de maintien en position basse qui est alors fixe.

6. Instrument selon l'une des revendications 1 à 5, caractérisé en ce que le déplacement maximal de la matrice supérieure (19) par rapport au dispositif (18) de maintien en position basse est limité vers le bas par des butées (28, 29; 24, 30).

7. Instrument selon l'une des revendications 1 à 6, dans lequel une série d'agrafes repose sur un coulisseau en pouvant être y déplacée par glissenent, caractérisé en ce que la matrice inférieure (15) est décalée vers le bas par rapport au coulisseau (11) et en ce que la hauteur h du décalage et le diamètre d de la matière d'agrafe sont liés par la relation suivante: 0,25 d ≦ h ≦ d.

8. Instrument selon l'une des revendications 1 à 7, composé d'une première partie d'instrument, prévue pour une utilisation répétée, et d'une deuxième partie d'instrument, reliée à la première de manière détachable et contenant le magasin, prévue pour une utilisation unique, caractérisé en ce que les deux matrices (15, 19) et le dispositif (18) de maintien en position basse sont eux aussi associés, en plus du magasin (10), à la deuxième partie d'instrument (32)

9. Instrument selon la revendication 8, caractérisé en ce que les deux parties d'instrument (31, 32) sont maintenues assemblées entre elles par une liaison à cliquet (35, 36).

10. Instrument selon l'une des revendications 1 à 9, caractérisé en ce que la dimension de la déformation d'agrafe est réglable de façon variable au moyen d'une butée réglable (37), en particulier d'une butée limitant le déplacement de réglage de la poigne pivotante (5b).
